# EUROPÄISCHE PATENTSCHRIFT

(11) **EP 0 656 769 B1**
(45) Veröffentlichungstag und Bekanntmachung des Hinweises auf die Patenterteilung: **17.12.1997**
(21) Anmeldenummer: 94916180.6
(22) Anmeldetag: 02.05.1994
(51) Int. Cl.: A61F 2/52

(54) **BRUSTPROTHESE**
BREAST PROSTHESIS
PROTHESE MAMMAIRE

(30) Priorität: 30.04.1993 DE 9306572 U
(43) Veröffentlichungstag der Anmeldung: 14.06.1995
(73) Patentinhaber: AMOENA Medizin-Orthopädie-Technik GmbH, D-83064 Raubling (DE)
(72) Erfinder: WILD, Helmut, D-83115 Neubeuern (DE)
(74) Vertreter: Gossel, Hans K., Dipl.-Ing.
(86) Internationale Anmeldenummer: EP9401381
(87) Internationale Veröffentlichungsnummer: WO9424964

(56) Entgegenhaltungen:
- EP-A- 0 392 960
- DE-A- 2 742 394
- DE-A- 2 802 375
- DE-A- 2 908 843
- DE-U- 9 114 512
- DE-U- 9 203 947
- DE-U- 9 306 572
- US-A- 3 858 248

## Beschreibung

Die Erfindung betrifft eine Prothese nach dem Oberbegriff des Patentanspruchs 1.

Bei einer derartigen Prothese wird der Tragstreifen in üblicher Weise auf der Brust der Trägerin befestigt. Der Tragstreifen selbst ist jedoch mit dem Rand der Brustprothese nur in seinem mittleren Bereich verbunden. Durch diese Art der Verbindung wird also durch Bewegungen der Brustprothese nur der mittlere Bereich des Tragstreifens belastet, so daß auf die Ränder des Tragstreifens keine ein abschälendes Lösen der Klebeverbindung verursachende Kräfte eingeleitet werden können. Bei Bewegungen der Brustprothese führt der Prothesenrand schwenkende oder kippende Bewegungen relativ zu dem Tragstreifen aus, wobei sich der Brustprothesenrand auf den Rändern des Tragstreifens abstützt und auf diese statt einer abziehenden oder einer abschälenden Bewegung eine andrückende Bewegung ausübt, weil die mittlere Verbindung eher wie eine schanierartige Verbindung wirkt.

Eine Prothese dieser Art ist aus der EP-A 0 392 960 bekannt. Bei dieser Prothese wird die Verbindung zwischen der Brustprothese und dem Tragstreifen mittels eine Klettverschlusses gewährleistet.

Aufgabe der Erfindung ist es daher, eine Prothese der eingangs angegebenen Art zu schaffen, die sich dauerhafter an der Brust einer Trägern befestigen läßt.

Erfindungsgemäß wird diese Aufgabe dadurch gelöst, daß die Verbindung der Brustprothese mit dem Tragstreifen gemäß Anspruch 1 unlösbar ausgebildet ist, wobei hier unter Unlösbarkeit eine Verbindung zu verstehen ist, die sich nur durch Zerstörung lösen läßt.

Die Verbindung zwischen dem Tragstreifen und dem Prothesenrand besteht zweckmäßigerweise aus im Abstand voneinander angeordneten Verbindungsflecken, die im mittleren Bereich des Tragstreifens angeordnet sind. Durch diese Art der punktförmigen Verbindung wird eine mittige Anbindung des Tragstreifens an die Brustprothese erreicht, die deren Beweglichkeit gegenüber dem Tragstreifen oder der Haftschicht noch verbessert und dadurch den guten Klebesitz der Haftschicht oder des Tragstreifens fördert.

Zweckmäßigerweise ist der Tragstreifen auf seiner der Brust abgewandten Seite mit konvex gekrümmten Randbereichen versehen. Diese Krümmungen verbessern das Abrollen des Prothesenrandes auf den Randbereichen des Tragstreifens.

Die günstige Haftwirkung der erfindungsgemäßen Prothese wird also dadurch bewirkt, daß die Verbindung zwischen dem Tragstreifen und dem Brustprothesenrand sich in dem mittleren Bereich des Tragstreifens befindet, so daß diese Verbindung bei Bewegungen der Brustprothese, bei denen sich die Brustprothese auf den Randbereichen des Tragstreifens abstützt, die mittige Verbindung im wesentlichen auf Zug beansprucht wird, was andererseits durch Abstützen der Prothese auf die Tragstreifenränder ein die Haftung des Tragstreifens auf dem Körper der Trägerin erhöhendes Andrücken der Tragstreifenränder zur Folge hat.

In weiterer Ausgestaltung der Erfindung ist vorgesehen, daß die Brustprothese auf ihrer Rückseite mit einer Aushöhlung versehen ist, in die ein Abdeckkissen aus faserigem und/oder textilem Material eingelegt ist, das an mindestens einer Stelle die verbindung zwischen dem Prothesenrand und dem Tragstreifen durchsetzt. Ein derartiges Abdeckkissen erhöht den Tragekomfort, weil sie verhindert, daß die Prothesenwand bzw. die die Prothese einfassende Folie direkt auf dem Körper aufliegt, was insbesondere beim Transpirieren unangenehm ist. Das Kissen saugt den Schweiß vom Körper ab und hält die Haut trocken. Die rückseitige Aushöhlung der Prothese erzeugt ein Luftkissen zwischen dem Körper und der Prothese, so daß bei Bewegungen der Prothese Luft aus dem Hohlraum verdrängt werden kann, die dann bei dem Ausströmen unter dem Prothesenrand unangenehme Geräusche verursachen kann. Dies wird ebenfalls durch das eingelegte Abdeckkissen dadurch verhindert, daß dieses an mindestens einer Stelle die Verbindung zwischen dem Prothesenrand und dem Tragstreifen durchsetzt.

Zweckmäßigerweise weist das Abdeckkissen einen Fortsatz auf, der den Spalt zwischen dem Prothesenrand und dem Tragstreifen im mittleren unteren Bereich des Tragstreifens durchsetzt. Sollte sich in der Höhlung Schweiß ansammeln, kann dieser durch die mittlere untere Öffnung austreten.

In weiterer Ausgestaltung der Erfindung ist vorgesehen, daß der innere Rand des Tragstreifens den Rand des Abdeckkissens zumindest teilweise übergreift, so daß das Abdeckkissen durch den Tragstreifen selbst in der Höhlung gehalten ist.

Ein Ausführungsbeispiel der Erfindung wird nachstehend anhand der Zeichnung näher erläutert. In dieser zeigt
- Fig. 1: eine Draufsicht auf die Rückseite einer ersten Ausführungsform einer Prothese,
- Fig. 2: eine der Fig. 1 entsprechende Ansicht einer zweiter Aus führungsform einer Prothese und
- Fig. 3: einen Schnitt durch die Prothesen gemäß den Linien A-A in den Figuren 1 und 2 in vergrößerter Darstellung.

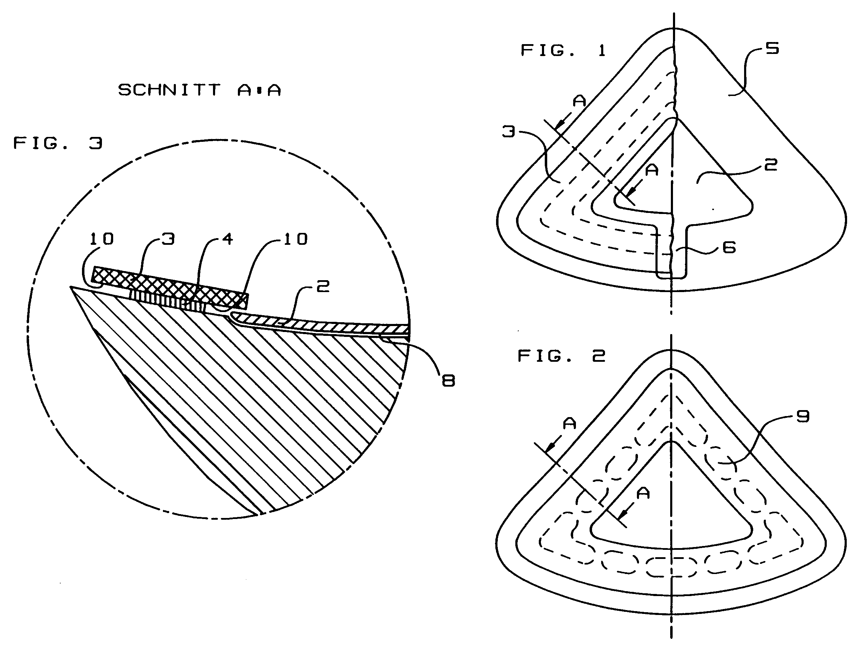

Aus Fig. 1 ist die Rückseite einer üblichen Brustprothese ersichtlich, die aus einem schalenförmigen Körper aus einer weichelastisch eingestellten, additionsvernetzenden Zwei-Komponenten-Silikon-Kautschuk-Masse besteht, der in Kunststoffolien eingeschweißt ist, die dessen Außenseite und dessen Innenseite abdecken. Die Brustprothese ist auf ihrer Rückseite mit einer flachen Aushöhlung versehen, in die ein Abdeckkissen 2 eingelegt ist. Die Brustprothese ist auf ihrer Rückseite längs ihres Randbereiches mit einem Tragstreifen 3 verbunden, der nur in seinem mittleren Bereich durch eine streifenförmige, dauerfeste Klebver bindung 4 mit dem Randbereich 5 der Brustprothese verbunden ist.

Der Tragstreifen 3 ist auf seiner Rückseite mit einer dauerklebrigen Schicht, beispielsweise einer Haft-Silikonschicht, versehen.

Die mittige Verbindung 4 ist unlösbar, so daß zum Anlegen und Abnehmen der Brustprothese jeweils ein Ankleben über die dauerklebrige Schicht des Haftstreifens 3 erfolgt.

Das Abdeckkissen 2 ist an seinem unteren Ende mit einem streifenförmigen Fortsatz 6 versehen, der einen Spalt zwischen dem Tragstreifen 3 und dem Prothesenrand 5 durchsetzt.

Der innere Rand des Tragstreifens 3 übergreift in der aus Fig. 3 ersichtlichen Weise den Rand des Abdeckkissens 2, so daß dieses ohne zusätzliche Haltemittel in der Aushöhlung 8 auf der Prothesenrückseite gehalten ist.

Die Ausführungsform nach Fig. 2 unterscheidet sich von der nach Fig. 1 dadurch, daß der mittige Verbindungsstreifen zwischen dem Tragstreifen 3 und dem Randbereich der Prothese nicht durchgehend ausgeführt ist, sondern aus im Abstand voneinander angeordneten Verbindungsflecken 9 besteht, die punktförmig oder länglich ausgebildet sein können. Die Verbindungsflecken können in den Eckbereichen winkelig ausgeführt sein.

## Patentansprüche

1. Prothese bestehend aus einer Brustprothese aus einem in seiner Konsistenz und Beweglichkeit dem natürlichen Brustgewebe angenäherten Material, wobei die Brustprothese vorzugsweise aus einem schalenförmigen Körper aus einer weichelastisch eingestellten, additionsvernetzenden 2-Komponenten-Silikon-Kautschuk-Masse besteht, der in Kunststoffolien eingeschweißt ist, und einem Tragstreifen (3), wobei die Brustprothese in ihrem Randbereich zumindest stellenweise durch eine Verbindung (4, 9) mit dem Tragstreifen (3) verbunden ist, der zur Befestigung auf der Brust der Trägerin mit einer dauerklebrigen Schicht versehen ist, und wobei sich die Verbindung (4, 9) nur im mittleren Bereich des Tragstreifens (3) befindet,
**dadurch gekennzeichnet,**
daß die Verbindung (4, 9) unlösbar ist.

2. Prothese nach Anspruch 1, dadurch gekennzeichnet, daß die Verbindung aus im Abstand voneinander angeordneten Verbindungsflecken (9) besteht.

3. Prothese nach einem der Ansprüche 1 oder 2, dadurch gekennzeichnet, daß der Tragstreifen (3) auf seiner der Brust der Trägerin abgewandten Seite mit konvex gekrümmten Randbereichen (10) versehen ist.

4. Prothese nach einem der Ansprüche 1 bis 3, dadurch gekennzeichnet, daß diese auf ihrer Rückseite mit einer Aushöhlung (8) versehen ist, in die ein Abdeckkissen (2) aus faserigem und/oder textilem Material eingelegt ist, das an mindestens einer Stelle die Verbindung zwischen dem Prothesenrand (5) und dem Tragstreifen (3) durchsetzt.

5. Prothese nach Anspruch 4, dadurch gekennzeichnet, daß das Abdeckkissen (2) mit einem Fortsatz (6) versehen ist, der den Spalt zwischen dem Prothesenrand und dem Tragstreifen (3) im mittleren unteren Bereich des Tragstreifens (3) durchsetzt.

6. Prothese nach Anspruch 4 oder 5, dadurch gekennzeichnet, daß der innere Rand des Tragstreifens (3) den Rand des Abdeckkissens (2) zumindest teilweise übergreift.

## Claims

1. Prosthesis, being a breast prosthesis made of a material which in terms of its consistency and mobility is similar to the natural breast tissue, the breast prosthesis preferably comprising a cup-shaped article which is made of a nonrigid, addition-crosslinking 2-component silicone rubber composition and which is welded into plastic films, and a support strip (3), the breast prosthesis in its edge area being connected at least in places to the support strip (3) via a connection (4, 9), which support strip (3) is provided with a permanently adhesive layer for securing it on the user's breast, and the connection (4, 9) being located only in the middle area of the support strip (3), characterized in that the connection (4, 9) is nonreleasable.

2. Prosthesis according to Claim 1, characterized in that the connection comprises connection spots (9) which are arranged at a distance from one another.

3. Prosthesis according to one of Claims 1 and 2, characterized in that the support strip (3) is provided with convexly curved edge areas (10) on its side directed away from the user's breast.

4. Prosthesis according to one of Claims 1 to 3, characterized in that this is provided on its reverse side with an indentation (8) into which a masking cushion (2) of fibrous and/or textile material is inlaid, which masking cushion (2) at at least one point passes through the connection between the prosthesis edge (5) and the support strip (3).

5. Prosthesis according to Claim 4, characterized in that the masking cushion (2) is provided with a continuation (6) which passes through the gap between the prosthesis edge and the support strip (3) in the middle, lower area of the support strip (3).

6. Prosthesis according to Claim 4 or 5, characterized in that the inner edge of the support strip (3) engages at least partially over the edge of the masking cushion (2).

## Revendications

1. Prothèse constituée d'une prothèse mammaire en un matériau dont la consistance et la mobilité approchent le tissu mammaire naturel, la prothèse mammaire étant constituée de préférence d'un corps en forme de coque en une masse de caoutchouc silicone à deux composants réticulés par addition, d'une élasticité molle, qui est soudé dans des feuilles de matière synthétique, et d'une bande porteuse (3), la prothèse mammaire étant reliée dans sa zone de bord au moins par endroits par un assemblage (4, 9) à la bande porteuse (3) qui est pourvue, en vue de la fixation sur le sein de la personne qui la porte d'une couche de colle permanente, et où l'assemblage (4, 9) se trouve seulement dans la zone centrale de la bande porteuse (3), caractérisé en ce que l'assemblage (4, 9) est indétachable.

2. Prothèse selon la revendication 1, caractérisée en ce que l'assemblage est constitué de pièces d'assemblage (9) disposées à une certaine distance les unes des autres.

3. Prothèse selon l'une des revendications 1 ou 2, caractérisée en ce que la bande porteuse (3) est pourvue sur son côté éloigné du sein de la personne qui la porte de zones de bord (10) courbées de manière convexe.

4. Prothèse selon l'une des revendications 1 à 3, caractérisée en ce que celle-ci présente sur son côté arrière un creux (8) dans lequel est placé un coussin de recouvrement (2) en une matière fibreuse et/ou textile, qui traverse à au moins un endroit l'assemblage entre le bord (5) de la prothèse et la bande porteuse (3).

5. Prothèse selon la revendication 4, caractérisée en ce que le coussin de recouvrement (2) est pourvu d'un prolongement (6) qui traverse la fente entre le bord de la prothèse et la bande porteuse (3) dans la zone centrale inférieure de la bande porteuse (3).

6. Prothèse selon la revendication 4 ou 5, caractérisée en ce que le bord intérieur de la bande porteuse (3) recouvre le bord du coussin de recouvrement (2) au moins partiellement.
